(19) Europäisches Patentamt
European Patent Office
Office européen des brevets

(11) **EP 3 048 884 B1**

## (12) EUROPEAN PATENT SPECIFICATION

(45) Date of publication and mention
of the grant of the patent:
**02.01.2019 Bulletin 2019/01**

(21) Application number: **14781831.4**

(22) Date of filing: **23.09.2014**

(51) Int Cl.:
*A01N 25/34* (2006.01)          *A01N 25/30* (2006.01)
*A01N 31/14* (2006.01)          *C09D 5/14* (2006.01)
*C09D 171/02* (2006.01)          *C11D 3/00* (2006.01)
*C11D 17/04* (2006.01)          *A01P 1/00* (2006.01)
*C11D 1/00* (2006.01)          *A61L 2/18* (2006.01)
*G02B 27/00* (2006.01)

(86) International application number:
**PCT/EP2014/070280**

(87) International publication number:
**WO 2015/044152 (02.04.2015 Gazette 2015/13)**

(54) **ANTIMICROBIAL COMPOSITION AND TISSUE CONTAINING IT**

ANTIMIKROBIELLE ZUSAMMENSETZUNG UND DEREN ENTHALTENDE GEWEBE

COMPOSITION ANTIMICROBIENNE ET TISSU LE CONTENANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.09.2013 EP 13306304**

(43) Date of publication of application:
**03.08.2016 Bulletin 2016/31**

(73) Proprietor: **Satisloh AG**
**6340 Baar (CH)**

(72) Inventor: **CADET, Mamonjy**
**F-94220 Charenton Le Pont (FR)**

(74) Representative: **Jacobacci Coralis Harle**
**32, rue de l'Arcade**
**75008 Paris (FR)**

(56) References cited:
**WO-A1-94/14945          WO-A1-96/17918
WO-A1-98/01524          WO-A1-2013/013929
WO-A1-2013/053406          GB-A- 1 604 859
JP-A- H11 100 600          US-A- 3 043 697
US-A- 5 994 286          US-A1- 2012 019 767**

**Description**

FIELD OF THE INVENTION

The present invention relates to a composition comprising a specific combination of a non ionic surfactant with poly(oxyalkylene) units and an alcohol component having good antimicrobial and antifogging properties.

The present invention also relates to an aqueous solution comprising said antimicrobial and antifogging composition.

The present invention also relates to antifogging tissues impregnated with said aqueous solution and an optical article comprising a main surface coated with a temporary antifog and/or antimicrobial coating obtained by wiping said main surface with the above tissue.

DESCRIPTION OF THE PRIOR ART

Very numerous supports, such as plastic materials and glass, suffer as a drawback from becoming covered with fog when their surface temperature decreases below the dew point of ambient air. This is especially the case with the glass that is used to make glazing for transportation vehicles or buildings, glasses for spectacles, lenses, mirrors, and so on. The fogging that develops on these surfaces leads to a decrease in transparency, due to the diffusion of light through water drops, which may cause a substantial discomfort.

To prevent any fog formation in very damp environments, that is to say the condensation of very little water droplets on a support, it has been suggested to apply hydrophilic coatings onto the outer surface of such support, with a low static contact angle with water, preferably of less than 50°, more preferably of less than 25°. Such permanent antifog coatings do act as sponges toward fog and enable the water droplets to adhere to the surface of the support by forming a very thin film that gives an impression of transparency. These coatings are generally made of highly hydrophilic species such as sulfonates or polyurethanes.

Commercially available products comprise several micrometer-thick hydrophilic layers.

As a rule, when the thickness of the coatings is high (several microns), these coatings, as a consequence of water absorption, do swell, soften and become mechanically less resistant.

As used herein, a permanent antifog coating is intended to mean a coating which hydrophilic properties result from hydrophilic compounds permanently bound to another coating or support. The application EP 1324078 describes a lens coated with an abrasion-resistant coating and a multilayered antireflective coating comprising layers with high and low refractive indexes alternating with each other, amongst which the outer layer is a low refractive index layer (1.42-1.48) of from 5 to 100 nm thickness forming an antifog coating consisting in a hybrid layer with a static contact angle with water of less than 10°, obtained through vacuum deposition of both simultaneously an organic compound and silica or of silica and alumina, that is to say through coevaporation of these various components. The antifog coating preferably comprises from 0.02 to 70% by weight of the organic compound relative to the coating total weight, and typically from 6 to 15% by weight, according to the examples.

Said organic compound comprises one hydrophilic group and one reactive group, for example a trialkoxysilyl group having from 3 to 15 carbon atoms, and has preferably a molecular weight ranging from 150 to 1500 g/mol. Some preferred compounds possess a polyether backbone, especially one polyoxyethylene and one reactive group on each end of the molecule. Preferred compounds include polyethylene glycol glycidyl ether, polyethylene glycol monoacrylate and N-(3-trimethoxysilylpropyl)gluconamide.

The antifog coating therefore comes as a silica-based layer (or a silica + alumina-based layer) incorporating one hydrophilic organic compound. However, its antifog character does change over time, and it can be observed a stepwise deterioration of the antifogging properties. When becoming too low, they may be restored through a "washing treatment" of the antifog film, particularly a plasma-mediated treatment.

In the practice, the coevaporation method of the application EP 1324078 is very complicated to implement. It would be preferable to have a method for making an antifog coating without carrying out any coevaporation process.

Antifogging properties may also be obtained by applying temporary solutions commercially available as sprays or towelettes, onto spectacle glasses comprising as the outer layer an antisoiling coating (hydrophobic and oleophobic), often considered as essential when ophthalmic glass is provided with an antireflective coating. They make it possible to obtain the antifogging property on a short period of time. The ease of soil removal aspect that is given to the antisoiling coating is preserved, but after a couple of wiping operations, the antifogging property is significantly altered. Indeed, temporary solutions comprise materials that are hydrophilic in nature with poor interactions with the antisoiling coating hydrophobic surface, so that after a few wiping operations, these hydrophilic materials are removed.

A more interesting solution consists in making an antifog coating by applying a temporary hydrophilic solution onto the surface of an antifog coating precursor coating, which represents an alternative to permanent antifog coatings.

The application EP 1275624 describes a lens coated with a hard, inorganic, hydrophilic layer based on metal oxides and silicon oxide. Its hydrophilic nature and the presence of nanosized concave portions on the surface thereof enable to impregnate a surfactant and to retain the same adsorbed over a long period of time, thus maintaining an antifog effect for several days. However, an antifog effect can also be observed in the absence of any surfactant.

The patent application WO 2011/080472 describes a glass for spectacles comprising a substrate provided with a coating comprising silanol groups on the surface thereof and, directly contacting this coating, an antifog coating precursor coating, wherein the antifog coating precursor coating:

- is obtained through the grafting of at least one organosilane compound possessing:

    o a polyoxyalkylene group comprising less than 80 carbon atoms, and
    ∘ at least one silicon atom carrying at least one hydrolyzable group,

- has a thickness lower than or equal to 5 nm,
- has a static contact angle with water of more than 10° and of less than 50°.

The temporary hydrophilic solution which is preferably deposited to provide this surface with antifogging properties is the commercially available solution Defog it™ .

The antifogging properties, especially the durability of the antifogging effect associated with the glass precursor coating described in the patent application WO 2011/080472, are very satisfactory.

However, it is desirable to improve the properties of the temporary antifogging coating of the lenses for spectacles, which are described in the patent application WO 2011/080472.

WO 98/0152 relates to an antimicrobial cleaning composition containing surfactant and substituted aromatic alcohols or phenols.

The document WO 94/14945 discloses antimicrobial cleaning composition containing a non-ionic or anionic surfactant and an alcohol.

WO96/17918 describes a desinfecting compositio comprising an ethoxylated nonionic surfactant, an anionic surfacatnt, anionic polymer and an antimicrobial agent.

US 5,994,286 describes an antimicrobial cleaning composition comprising a surfactant, triclosan and tocopherol.

GB 1 604 859 discloses antiparasitic composition comprising monohydric alcohol and a polyoxyethylene derivative.

WO 2013/013929 describes an optical article comprising : - a precursor coating of an antifog cotaing obtained through the grafting of one organosilane compound having a polyoxyalkylene group and at least one silicon and - a film obtained by applying onto the precursor coating a surfactant-containing composition.

US 2012/019767 discloses a spectacle lens comprising a grafted anti-fog coating comprising silanol groups bearing a polyoxyalkylene group.

US 3 043 697 describes a photographic silver halide amulsion comprising monohydric or dihydric phenols as anti-fogging agents.

HP H11 100600 dsicloses an antifogging detergent composition comprising fluorinated surfactant and an antimicrobial agent.

Therefore, there is still a need to provide a novel composition intended to form a temporary film on a surface of an optical article, having good antifogging properties, while preventing the proliferation of germs onto said optical article. In particular, temporary compositions with both antimicrobial and antifogging propeties are sought after, which would last longer over time and/or under mechanical stresses, while preserving an acceptable ease of soil removal.

SUMMARY OF THE INVENTION

An object of the invention is therefore to remedy the above drawbacks, by seeking to develop an antifogging composition or temporary coating having significantly improved antimicrobial properties versus the temporary hydrophilic compositions of the prior art, while having also good or enhanced antifogging property durability, over time and/or under mechanical stresses.

The invention therefore relates to an antimicrobial composition such as described in the set of claims.

Preferably, the composition of the invention also exhibits antifog activity, when it is applied in particular on an optical article surface, such as spectacle lens surface.

The invention also relates to an aqueous solution comprising by weight relative to the total weight of the preparation at least 50%, preferably 60% of water and at least 10% of the antimicrobial and antifogging composition as defined above.

In a first embodiment, it is also provided a dry microfiber tissue obtained by impregnation with an aqueous solution as defined above, of microfiber tissue comprising microfibers made of polymers comprising polyester units and polyamide units, followed by drying said microfiber tissue so as to remove solvents present in the aqueous solution, the dry microfiber tissue comprising, by weight, relative to the total weight of said dry microfiber tissue, less than 5 % of solvents.

In a second embodiment, it is provided a non woven wet tissue whose structure comprises a hydrophilic polymer, preferably a hydrophilic polymer comprising cellulosic units, and a hydrophobic polymer, said tissue being impregnated by an aqueous solution as defined above.

The invention further relates to an optical article comprising a substrate having at least one main surface coated with a first coating and, directly contacting this first coating, a precursor coating of an antifog coating, characterized in that the coating precursor of the antifog coating:

- is obtained through the grafting of at least one organosilane compound having:

    ◦ a polyoxyalkylene group comprising preferably less than 80 carbon atoms, more preferably less than 40 carbon atoms, and optionally
    ◦ at least one silicon atom bearing at least one hydrolyzable group,

- and is further coated with a film obtained by applying onto said precursor coating a temporary film comprising the antimicrobial and antifog composition as defined above.

According to the invention, there is also provided a method for imparting antimirobial and antifogging properties to an optical article having at least one main surface, comprising the application onto said main surface of said temporary antimicrobial and antifog composition as defined above, generally in a film form.

BRIEF DESCRIPTION OF THE DRAWING

The present invention will be described in more detail by referring to the appended drawings, wherein:

- Figure 1 shows the variation of the logarithmic value observed for five strains *Pseudomonas aeruginosa* DSM 1128 (PA), *Staphylococcus aureus* DSM 799 (SA), *Escherichia coli* DSM 1576 (EC), *Candida albicans* DSM 1386 (CA) and *Aspergillus brasiliensis* DSM 1988 (AB) inoculated on a naked tissue CEMOI™, depending on the contact time (at day 7, 14, 21 and 28);
- Figure 2 shows the variation of the logarithmic value observed for the same five strains as figure 1 (PA, SA, EC, CA, AB) inoculated on a tissue CEMOI™ impregnated with 1% of phenoxyethanol, depending on the contact time (at day 7, 14, 21 and 28);
- Figure 3 shows the variation of the logarithmic value observed for the same five strains as figure 1 (PA, SA, EC, CA, AB) inoculated on a tissue CEMOI™ impregnated with 30% of a fluoroalkyl-polyoxyalkylene surfactant (CAPSTONE FS 3100™) depending on the contact time (at day 7, 14, 21 and 28); and
- Figure 4 shows the variation of the logarithmic value observed for the same five strains as figure 1 (PA, SA, EC, CA, AB) inoculated on a tissue CEMOI™ impregnated with 30% of a fluoroalkyl-polyoxyalkylene surfactant (CAPSTONE FS 3100™) and 1% of phenoxyethanol depending on the contact time (at day 7, 14, 21 and 28).

DETAILED DESCRIPTION OF THE INVENTION AND PREFERRED EMBODIMENTS

The terms "comprise" (and any grammatical variation thereof, such as "comprises" and "comprising"), "have" (and any grammatical variation thereof, such as "has" and "having"), "contain" (and any grammatical variation thereof, such as "contains" and "containing"), and "include" (and any grammatical variation thereof, such as "includes" and "including") are openended linking verbs. They are used to specify the presence of stated features, integers, steps or components or groups thereof, but do not preclude the presence or addition of one or more other features, integers, steps or components or groups thereof. As a result, a method, or a step in a method, that "comprises," "has," "contains," or "includes" one or more steps or elements possesses those one or more steps or elements, but is not limited to possessing only those one or more steps or elements.

As used herein, an "antifog coating" is intended to mean a coating which, when a transparent lens substrate coated with such coating is placed under conditions generating fog onto said substrate being devoid of said coating, enables to immediately attain a visual acuity > 6/10 for an observer looking through a coated lens at a visual acuity scale located at a distance of 5 meters. Several tests to evaluate the antifogging properties of a coating are described in the experimental section. Under fog generating conditions, antifog coatings may either not present fog on their surface (ideally no visual distortion, or visual distortion but visual acuity > 6/10 under the hereabove mentioned measurement conditions), or may present some fog on their surface but yet enable, despite the vision perturbation resulting from fog, a visual acuity > 6/10 under the hereabove mentioned measurement conditions. A non-antifog coating does not allow a visual acuity > 6/10 as long as it is exposed to conditions generating fog and generally presents a condensation haze under the hereabove mentioned measurement conditions.

As used herein, an "antifog optical article" is intended to mean an optical article provided with an "antifog coating" such as defined hereabove.

Also unless otherwise indicated, the indication of an interval of values « from X to Y » or "between X to Y", according to the present invention, means as including the values of X and Y.

As previously mentioned, an object of the present invention relates to an antimicrobial and optionally antifogging composition comprising:

(a) at least a non ionic surfactant which is a fluoroalkyle polyethoxylated surfactant of formula $F(CF_2)_S-(CH_2-CH_2O)H$ (VIII), wherein "s" is an integer and is equal or less than 10 and "r" is also an integer and is ranging from 1 to 16.

The antimicrobial composition according to the invention also comprises (b) at least an alcohol compound of low molecular weight equal or less than 500 g/mol, which is phenoxyethanol, wherein the weight ratio R of the non ionic surfactant/alcohol compound is such that $2.5 \leq R \leq 200$, and preferably the content of the at least alhocol compound by weight, relative to the total weight of the bacteriocidal and fungicidal composition is ranging from 0.01% to 5%, preferably from 0.01% to 2%.

As used herein, "antimicrobial composition" means a composition having activity against bacteria and/or fungus and/or yeast and/or moulds; in particular, this includes biocide, bacteriostatic, bateriocidal, yeasticidal, antifungal, fungicidal, fungistatic and/or repellent activity.

As used herein, "a non ionic surfactant comprising a hydrophilic unit" means that the non ionic surfactant carries at least a hydrophilic group. "Hydrophilic groups" mean according to the present invention combinations of atoms which are suitable to link with water molecules, especially by hydrogen bond. Generally, there are polar organic groups.

As used herein, "a non ionic surfactant comprising a hydrophobic unit" means that the non ionic surfactant carries at least a hydrophobic group. "Hydrophobic groups" mean according to the present invention, combinations of atoms which are not suitable to link with water molecules, especially by hydrogen bond. Generally, there are non polar organic groups.

Surprinsingly, it has been found according to the invention that the specific combination of a non ionic surfactant with an alcohol as defined above, enables to obtain enhanced antimicrobial activity; the temporary film or coating obtained once it is applied on a main surface of an optical article thanks to an impregnated tissu of this specific combination, exhibits both excellent antifogging properties and improved antimicrobial activity.

More surprisingly, it has been found that there is synergistic or at least an enhanced effect of the antimicrobial property of the specific combination of the coumpounds of the invention as compared with antimicrobial property of each component taken separately, while having also good or improved antifogging property durability over time and/or under mechanical stresses.

This is of particular interest, since it allows to use low concentration of alcohol compound in the final composition.

Consequently, the present invention enables to reduce, inibit or delay growth of microorganisms, such as bacteria, yeast or fungus, or alternatively kill or destroy such mircoorganims, in particular on a main surface of an optical article, such as transparent optical article, once it is applied thereon.

Advantageously, the weight ratio ratio R of the non ionic surfactant/alcohol compound is such that $20 \leq R \leq 50$, preferably ratio R of the non ionic surfactant/alcohol compound is such that $20 \leq R \leq 40$.

By using specific ratio of the two specific compounds of the invention, it is possible to modulate the antimicrobial properties and to prevent and control bacterial/germ adhesion on an optical article, such as spectacle lens.

Preferably, the content of the at least alcohol compound by weight, relative to the total weight of the antimicrobial composition is ranging from 0.01% to 5%, generally from 0.01% to 2%, more preferably from 0.25% to 2%, and typically is ranging from 0.5 to 1.5%.

According to the invention, the alcohol is phenoxyethanol.

According to the present invention, the non ionic surfactant, which may be used as an antifogging agent, may have an Hydrophilic Lipophilic Balance equal or higher than 5, preferably equel or less than 18, in particular equal or less than 16 and typically equal or less than 15, so as to obtain good antifogging properties.

The method of calculating defined in the publication of W.C.Griffin, J. Cosm Ploughshare. Chem. 1954(vol. 5), pages 249-56, namely HLB= 20 X Mh/M, formula in which Mh is the molecular mass of the hydrophilic portion of the molecule and M is the total molecular mass of the molecule giving a result on a scale from 0 to 20, may be used. A value of HLB of 0 calculated with the method of Griffin corresponds to a completely lipophilic/hydrophobic molecule and, a value of 20 corresponds to a completely hydrophilic/lipophobic molecule.

The non ionic surfactant is preferably liquid surfactant, that is to say the melting temperature is less than 35°C at atmospherique pressure.

Preferably, it does not present a phenomenon of evaporation too marked, or unpleasant odor, does not confer on the tissue a fatty touch, nor does not modify its aspect unfavourably, does not generate optical or cosmetic defects on treated surface, and is not toxic, since the tissue of the invention are generally handled with naked hands and/or near the eye, in particular if they are intended to treat an

Preferably, the content of the non ionic surfactant by weight relative to the total weight of the antimicrobial composition is at least 20%, at least, 25%, at least 30%, at least 35%, at least 40%, at least 45%, at least 50%, at least 55%, at least 60%, at least 65%, at least 70%, at least 75%, at least 75%, at least 80%, at least 85%, at least 90%, at least 95%, at least 99%, for instance 99,99%.

According to an emboiment, the content of the non ionic surfactant by weight relative to the total weight of the antimicrobial composition is for instance ranging from 10 to 40%, preferably from 20 to 30%.

According to another embodiment, the content of the non ionic surfactant by weight relative to the total weight of the antimicrobial composition is for instance ranging from 80 to 99.99%, preferably from 95 to 99,99%, and typically from 98 to 99,99%.

According to the invention, the non ionic surfactant is selected from fluoroalkyl-polyoxyalkylenes of formula $F(CF_2)_s$-$(CH_2$-$CH_2O)_r$H (VIII), in which s and r are integers such as r is ranging from 1 to 16 and s is equal or less than 10.

Among these fluorinated surfactants, the following products may be used :Capstone® FS 3100 (n=4.7), Capstone® FS30 (n=6.9), Capstone® FS 31(n=4,9), Capstone® FS 34 (n=6,8), Masurf FS 1700 (n=4,9), Masurf FS 1800 (n=4,3), Masurf FS 2800 (n='4,2), Masurf FS2900 (n=5,7), Zonyl® FSO 100 (n=5) and Zonyl® FSN 100 (n=5-6).

Capstone® FS 3100 is a surfactant comprising a mixture of compounds having variable lengths of polyethoxylated chains corresponding to the formula $F(CF_2)_s$-$(CH_2$-$CH_2O)_r$H (VIII) in which more than 90% by weight corresponds to the fraction s=6 , r being an integer ranging from 1 to 14. Capstone® FS3100 contains undetectable contents of compounds of formula (VIII) by HPLC in which s is higher than 6. It is biodegradable.

Zonyl® FSO 100 (HLB = 9,1), marketed by Dupont, is a mixture of compounds of formula $F(CF_2)_s$-$(CH_2$-$CH_2O)_r$H (VIII) in which s has the following values : 6, 8 and 10 in the respective mass proportions of about 65%, 30%, 5% and r is an integer ranging from 3 to 13.

Other surfactants like trisiloxane-Polyethoxylated (Coatosil 77™ obtainable from Momentive) (n=5.5) can be used.

The invention also relates to an aqueous solution comprising by weight relative to the total weight of the preparation at least 50%, preferably at least 60%, much better at least 75% of water and at least 10%, preferably at least 20%, typically at least 25% of the antimicrobial and antifog composition as defined above. Generally, the antimicrobial and antifog composition presents in the aqueous solution comprises, by weight relative to the total weight of the antimicrobial antifog composition, from 80% to 99.99%, preferably from 95% to 99,99%, and typically from 98% to 99,99% of the non ionic surfactant and from 0.01% to 2% of the alcohol compound of low molecular weight equal or less than 300 g/mol, which is phenoxyethanol.

The aqueous solution may also comprise a monofunctional alcohol and a difunctional alcohol, said monofuctional alcohol having preferably a lower molecular weight than said difunctional alcohol.

The monofunctional alcohol comprises one single hydroxy group, typically ethanol or isopropyl alcohol. The difunctional alcohol comprises only two hydroxy groups. An example of a particularly preferred difunctional alcohol is propylene glycol (propane-1, 2-diol).

This aqueous solution may be used as a bath in which a tissue or cloth is plunged so as to impregnate it and the tissue is directly used to confer antifogging and antimicrobial properties to the optical article coated with the precursor coating by wiping it with said tissue or cloth.

The tissue may be non woven wet tissue or dry microfiber tissue.

In one embodiment, the invention relates to the non woven wet tissue whose structure comprises a hydrophilic polymer, preferably a hydrophilic polymer comprising cellulosic units, and preferably a hydrophobic polymer, said tissue being impregnated by an aqueous solution as defined above.

An exemple of such a tissue is the tissue "wetlaid" manufactured by the Ahlstrom company.

A preferred hydrophilic polymer is a polymer comprising cellulosic units.

In another embodiment, the invention relates to a dry microfiber tissue impregnated by a composition as described before, wherein said dry microfiber tissue comprises by weight, relative to the total weight of said dry microfiber tissue, less than 5 % of a solvent.

In another embodiment, the invention also relates to a dry microfiber tissue (ie: which is dry to the touch since the solvent, such as water, has been evaporated) obtained for examples by impregnation with an aqueous solution as defined above, of a microfiber tissue, preferably comprising microfibers made of polymers comprising polyester units and polyamide units, followed by drying said microfiber tissue, so as to remove solvents present in the aqueous solution, the dry microfiber tissue comprising, by weight, relative to the total weight of said dry microfiber tissue, less than 5 % of solvents.

As known in the art, a microfiber tissue or cloth is made of microfibers. A microfiber is a fiber with less than 1.3 Decitex (Dtex) per filament, preferably less than 1 Decitex per filament. Decitex is a measure of linear density and is commonly used to describe the size of a fiber or filament. Ten thousand meters of a 1-decitex fiber weighs one gram. In a microfiber tissue, fibers are combined to create yarns, which are knitted or woven in a variety of constructions.

An example of a preferred microfiber tissue comprising microfibers made of polymers comprising polyester units and polyamide units is the CEMOI™ tissue (manufacturer: KB SEIREN Company - retailer: Facol) whose composition is polyester 70% / Nylon™ 30% and that is commonly used for cleaning lenses.

The purpose of the drying step in the preparation of the dry microfiber tissue is to remove solvents (water, optionally alcohol) present in the aqueous solution. It is generally a heating step. The heating step preferably comprises heating at a temperature ranging from 60°C to 200°C, more preferably from 80°C to 150°C, and more preferably around 120°C. Dry microfiber tissue comprises by weight, relative to the total weight of the tissue less than 5% of solvent. A value less than 5% means less than 4.5%, 4%, 3.5%, 3%, 2.5%, 2%, 1.5%, 1%, 0.8%, 0.5%, 0.2% and 0.1%.

After the heating step, the microfiber tissue comprising microfibers made of polymers comprising polyester units and polyamide units is dry and the weight content of the antimicrobial antifogging composition impregnating said microfiber tissue preferably ranges from 10% to 45%, more preferably 14% to 40 %, even better from 20 to 40% and optionally from 20% to 30% relative to the total weight of the dry impregnated microfiber tissue (tissue and composition).

It has been determined that, surprisingly, the dry microfiber tissue comprising microfibers made of polymers comprising polyester units and polyamide units having been impregnated by the above aqueous solution is still able to remove smudges from the surface of optical articles, while providing at the same time antifogging and antimicrobial properties with long lasting effect.

The composition of the present invention reduces the static contact angle with water of the surface of the optical article, especially of a spectacle lens. The antifog antimicrobial temporary coating or film thus obtained on the optical article surface preferably has a static contact angle with water lower than or equal to 10°, more preferably lower than or equal to 5°.

An immediately operational temporary antifog and antimicrobial film is obtained as soon as the antimicrobial antifogging composition of the invention comprising the spcecific combination and ratio of a non ionic surfactant with an alcohol compound, is applied, which represents an advantage of the invention. Thus, it is not necessary to apply many times a surfactant solution to score the antifogging effect, as is the case with products of the prior art.

In addition, the antifogging and antimicrobial effect provided by the antifog antimicrobial composition is long-lasting over time. In the experimental section, the synergic antimicrobial activity of the antimicrobial antifogg composition of the present invention is shown.

The present invention also relates to a method for imparting antifog and antimicrobial properties to an optical article, preferably a lens for spectacles, comprising the application of the antimicrobial and antifogging composition previously defined, which is preferably a liquid solution (aqueous solution comprising said antimicrobial and antifogging composition), onto a main surface of said optical article, and more preferably using a tissue or cloth impregnated by theantimicrobial and antifogging composition such as described hereabove, and especially the non woven wet tissue or the dry microfiber tissue as described above.

Preferably, the main surface of the optical article onto which said composition is applied has preferably a static contact angle with water of 90° or less, more preferably of more than 10° and of less than 50°. Said main surface is generally the surface of a coating applied on the substrate of the optical article, e.g. a precursor coating of an antifog coating. Preferably, said main surface is not the surface of a hydrophobic and/or oleophobic coating. Said main surface can be the uncoated surface of the optical article's substrate.

In the present disclosure, a lens does possess antifogging properties if it successfully passes the breath test. For this test, the tester places the lens to evaluate at a distance of about 2 cm from his mouth. The tester for 3 seconds blows his breath onto the exposed surface of the lens. The tester can visually observe the presence or the absence of a condensation haze/distorsion.

A lens is considered as having antifogging properties if it inhibits the haze effect resulting from the fog at the end of the breath test (but it does not necessarily represent an antifog lens within the meaning of claim 1, because it may possibly present a visual distorsion leading to a visual acuity <6/10).

Therefore, the method of the invention generally enables to provide antifogging and antimicrobial properties to any type of optical article, preferably lenses for spectacles, whether the article has an antifog coating precursor coating, or not. The method is especially recommended for treating bare lenses or lenses just coated with an abrasion-resistant coating, preferably of the polysiloxane-containing type.

In particular, the present invention relates to an optical article, preferably a lens for spectacles, comprising a substrate having at least one main surface coated with a first coating and, directly contacting this first coating, a precursor coating of an antifog coating, characterized in that the coating precursor of the antifog coating:

- is obtained through the grafting of at least one organosilane compound having:

o a polyoxyalkylene group comprising preferably less than 80 carbon atoms, more preferably less than 40 carbon atoms, and optionnaly

o at least one silicon atom bearing at least one hydrolyzable group,

- and is further coated with a film obtained by applying onto said precursor coating a temporary film comprising an antimicrobial and antifog composition of the present invention such as claimed.

The present inventio lastly relates to a method method for imparting antifog properties to an optical article having at least one main surface, comprising the application onto said main surface of a temporary antimicrobial and antifog film such as previously described.

Preferably, the application onto said main surface comprises wiping said main surface with a tissue, whose structure comprises a hydrophilic polymer and a hydrophobic polymer, said tissue being impregnated with said temporary antimicrobial and antifog composition.

The antifog and antimicrobial coating is temporary but easily renewable, since it just has to be performed a new application of the composition of the invention when there are not sufficient surfactant and alcohol compounds adsorbed onto the surface of the antifog coating precursor onto the opticle article surface anymore. The latter therefore remains "activable" in all circumstances.

Such optical articles can be manufactured according to any one of the methods disclosed in WO 2011/080472.

The following examples illustrate the invention in a more detailed yet non limiting way.

EXAMPLES

The analyzed samples are naked CEMOI™ tissue (reference sample 1) or CEMOI™ tissues impregnated either with an alcohol compound (reference sample 2), or a non ionic surfactant (reference sample 3) or with both an alcohol and a non ionic surfactant (sample 4 according to the invention), wherein five strains *Pseudomonas aeruginosa* DSM 1128 (PA), *Staphylococcus aureus* DSM 799 (SA), *Escherichia coli* DSM 1576 (EC), *Candida albicans* DSM 1386 (CA) and *Aspergillus brasiliensis* DSM 1988 (AB) have been inoculated. The variation the logarithmic value is measured according to the contact time: 7, 14, 21 and 28 days.

1. Testing protocol

*1.1 Cleanliness control*

The preliminary control of cleanliness enables to determine the initial contamination rate of the analyzed samples.

1 cm$^2$ of tissue is cut and 9mL of a neutralizing diluent (AEB111279 batch: 312005) is added. The tissue is then mixed with a vortexer during 30 seconds. The technique of setting in culture used is surface inoculation. The reading of the colonies is carried out after 72h incubation at 32.5 C +/- 2,5°C or the bacteria and at 25 C+/- 2,5°C for yeasts and moulds.

*1.2 Repeated tests*

Tripicate tests are carried out for each sample at each time of contact.

*1.3 Used strains*

Five strains have been independently used:

- *Pseudomonas aeruginosa* DSM 1128 (Pa)
- *Staphylococcus aureus* DSM 799 (SA)
- *Escherichia coli* DSM 1576 (EC)
- *Candida albicans* DSM 1386 (CA)
- *Aspergillus brasiliensis* DSM 1988 (AB)

*1.4 Contact time*

The launching of the analyses takes place at D0 (day 0).

Times of contact applied are: 7 days (J7), 14 days (J14), 21 days (J21) and 28 days (J28).

*1.5 Inoculation*

The germs are prepared in a suitable nutrient medium (NB) diluted with 1/500th.

At DO, all the samples are inoculated so as to obtain a germ final concentration ranging from $1.0.10^5$ to $1.0.10^6$ UFC/mL. The samples are covered with a PET film to prevent desiccation.

At each contact time, the following samples: D7, D14, D21 and D28 are again inoculated so as to obtain a final concentration ranging from $1.0.10^4$ and $1.0.10^5$ UFC/mL according to the following scheme:

- D0 samples : inoculated at D0 - then recovery of such samples in 10mL of SCDLP medium (Soybean-Casein Digest broth with Lecithin and. Polysorbate 80) at D0;
- D7 samples : inoculated at D0 - then recovery of such samples in 10mL of SCDLP medium at D7;
- D14 samples : inoculated at D0 and at D7- then recovery of such samples in 10mL of SCDLP medium et D14;
- D21 samples : inoculated at D0, D7 and D14- then recovery of such samples in 10mL of SCDLP medium at D21;
- D28 samples: inoculated at D0, D7, D14 and D21- then recovery of such samples in 10mL of SCDLP medium at D28.

*1.6 Incubation of the samples*

The samples are incubated at 20-25°C during all the period analysis.

*1.7 Recovery*

Once the samples have been inoculated and after each contact time, they are placed and then mixed in 10 mL of SCDLP medium. Dilutions are carried out and 100 $\mu$L of each dilution are transferred in duplicated in sterile Petri dishes. Then, 15 to 20 mL of TSA (Tryptic Soy Agar) for bacteria and 15 to 20 mL of SDC (Sabouraud Dextrose Agar with Chloramphenicol) for yeasts and moulds are added.

*1.8 Incubation and enumeration*

The enumeration of the colonies is carried out after 24 to 48 hours of incubation at 32.5°C +/-2.5°C for bacteria and yeast and after from 3 to 5 days of incubation at 25°C +/- 2.5 °C for moulds.

2. Results

*2.1 Control cleanliness of the samples*

The results are expressed in UFC per $cm^2$ for the based CEMOI™ tissues samples. The nutrient medium is TSA for bacteria and SDC for yeasts and moulds.

| CEMOI™ tissues tested | UFC/$cm^2$ |
|---|---|
| Bacteria | <100 |
| Yeasts/Moulds | <100 |

Thus, no contamination was detected on the 4 samples. The state of cleanliness of these samples is acceptable for continuation of the analysis.

*2.2 Calculation of data*

The number of UFC per sample is determined so as to calculate the logarithmic reduction value obtained a each contact time and for each analyzed strain.

Example of the calculation so to determine the UFC number per sample:

- Sample : sample 1 (naked tissue)
- Strain : *P. aeruginosa* (Pa)

- Contact time : D0
- Plated dilution : $10^{-2}$
- Plated volume : 100 $\mu$l (i.e: 0.1 mL)
- UFC number: 21/27/26/9/31/47, average number: 25.833
- Calculation : UFC average $\times$ (1/dilution rate) $\times$ (1/volume)

$$- \quad \text{Result} : 25.833 \times 100 \times 10 = 25833 \text{ UFC/sample}$$

Example of calculation for the logarithmic value : R

$$R = \log D0 - \log D_x \text{ with } D_x = \text{day 7, 14, 21 or 28}$$

- Sample : sample 1 (naked tissue)
- Strain : *P. aeruginosa* (Pa)
- Contact time : Variation logarithmic at D7

$$R(D7) = \log D0 - \log D7 = \log (25833) - \log (<100) = > 2.4$$

*2.3 Results*

Tables 1 to 4 below and figures 1 to 4 show the variation of the logarithmic value obtained after analysis of raw data for each sample according to the inoculated strain and to time contact. According to the invention, the following R values expressed a bactericidal or fungicidal activity:

- If $R \geq 2$, the compound tested has a bactericidal effect (PA, EC and SA),
- If $R \geq 1$, the compound tested has a fungicidal effect (AB), and
- If $R \geq 1$, the compound tested has a yeasticidal effect (CA),
- If $R > 0$, the compound tested kills the inoculated germs,
- If R is constant between two consecutive contact time, this means that there is no increase or decrease of the germs proliferation (i.e: bacteriostatic or fungistatic effect), and
- If $R < 0$, this means that the compound tested on the tissue sample increase the proliferation of the inoculated germ.

Reference sample 1: naked tissue

*Table 1*

|     | J7    | J14  | J21   | J28   |
| --- | ----- | ---- | ----- | ----- |
| PA  | >2,4  | 1,8  | -2,3  | -2,8  |
| SA  | >2,0  | 0,8  | >2,0  | >2,0  |
| EC  | 0,1   | -2,5 | -2,6  | -2,8  |
| CA  | -1,8  | -1   | -1,3  | -1,7  |
| AB  | 0,5   | 0,6  | 0,2   | 0,2   |

Reference sample 2: Tissue + 1% phenoxyethanol

*Table 2*

|  | J7 | J14 | J21 | J28 |
|---|---|---|---|---|
| **PA** | -0,2 | -1,8 | -2,4 | -0,4 |
| **SA** | 0,3 | 1,4 | 3,2 | >2,9 |
| **EC** | -1,5 | -2,1 | -2 | -1,9 |
| **CA** | -0,4 | -0,1 | -0,4 | 0,1 |
| **AB** | >2,3 | 0,8 | 1,3 | 0,8 |

Reference sample 3: Tissue + non ionic surfactant, i.e: Capstone F53100™ (30% by weight)

*Table 2*

|  | J7 | J14 | J21 | J28 |
|---|---|---|---|---|
| **PA** | -1,2 | -1,8 | -1,9 | -2,4 |
| **SA** | 2,1 | >2,6 | 2,1 | >2,6 |
| **EC** | -1,4 | -1,5 | 1,1 | >0,8 |
| **CA** | -0,3 | 0,9 | 0,9 | 1,5 |
| **AB** | 0,1 | >0,8 | 0,2 | >0,8 |

**Sample 4:** Tissue + non ionic surfactant i.e: Capstone FS3100™ (30% by weight) + 1% phenoxyethanol (according to the invention)

*Table 4*

|  | J7 | J14 | J21 | J28 |
|---|---|---|---|---|
| **PA** | -1,6 | >1,1 | >2,1 | >2,1 |
| **SA** | >2,2 | >2,2 | >2,2 | >2,2 |
| **EC** | 0,3 | >2,4 | >2,4 | >2,4 |
| **CA** | 1,1 | 1,3 | 2,7 | 1 |
| **AB** | 0,1 | >1,3 | 0,7 | 0,9 |

*2.4 Interpretation of the results*

Reference sample 1 1 is a untreated tissue. On this sample , the observed effect on germs come only from its structure, that is to say from the naked CEMOI™ tissue. As illustrated on fig.1 and table 1, the naked tissue has an effect on the tested germs:

- It has a bactericide effect on .S. aureus strains,
- It has no bactericidal or bacteriostatic effect on P. aeruginosa and E.coli,

It has no fungistatic on A.brasiliensis (the germ concentration is equivalent at various time contact),

- It has no effect yeasticidal effect on C. albicans.

Hence, it is shown that the naked tissue is, thanks to its structure and composition, bactericidal against S. aureus. However, it is also a nutritive source for E. Coli and P. aeruginosa since their growth is facilitated from 14 days of contact time.

Reference sample 2 with an alcohol compound has a fungsitatic effect on C. albicans (from D14). This effect is higher than the one observed with sample 1. Thus, the phenoxyethanol seems to reduce C. albicans profileration without however having a yeasticidal effect. Sample 2 has also a fungicidal effect on A. brasiliensisas strain from D14 (R is about 1 from D14 to D28). Therefore, the combination of the tissue with 1% of phenoxyethanol enables to obtain an effect on bacteria from 14 or 21 days of contact time. The phenoxyethanol does not enable to obtain a bacteriocidal effect on gram negative bacteria (PA and EC). It also however to improve the bacteriostatic effect (R tends towards 0) with a late reaction time (appears only after 21 days of contact time with the germs) forP aeruginosa.

Reference sample 3 with a non ionic surfactant has a bacteriocidal effect on S. aureus which is slightly higher than the one obtainded with a naked tissue (sample 1). After 14 days of contact time, the effect against *E. Coli* is improved. However, sample 3 does not have a bacteriocidal affect on P. aeruginosa between D7 and D28 (-1.2 <R<-2.4). The effect of the surfactant on the *C.albicans* yeast is much better than the one observed with sample 1. Indeed, a yeasticidal effect is observed with sample 2 after 14 days of contact time. Also, the combination surfactant + tissue anables to improve the effect on *A. brasiliensis* (R tends towards 1 from D14°.

Sample 4 according to the invention enables to obtain, contrary to the previous samples 1 to 3, a bacteriocidal effect on *S. aureus*, *E. coli*, *P.aeruginosa*, *C. albicans* after 14 days of contact. This sample allows to obtain a bacteriocidal activity on *P.aeruginosa* strain (not obtained with the previous samples). In addition, a fungicidal effect on *A. brasiliensis* is also observed from D14 of contact time *A fungicidal* effect on *A. brasilience* is also observed from 14 days of time contact. Therefore, the antimicrobial composition according to the invention shows a synergic effect as compared to the alcohol compound and the non ionic surfactant each taken separately. 2 log of logarithm reduction is obtained for all bacteria and 1 log is obtained for the yeast and the mould (from 14 days of contact to 21 days of contact).

The results of the experiments are resumed in the following table V:

* Reference samples

Table V

| Sample | Alcohol | Surfactant | Properties | Bacteria (gram -) | | Bacteria (gram +) | Fungus | Champignons | Comments |
|---|---|---|---|---|---|---|---|---|---|
| | | | | P. Aeruginosa | E. Coli | S. Aureus | C. Albican | A. Brasiliensis | |
| | | | | PA | EC | SA | CA | AB | |
| 1* | none | none | Bacteriostatic | KO | KO | OK | OK | OK | Bacteristatic effect on SA and AB |
| | none | none | Bacteriocidal | KO | KO | OK | KO | KO | Bacteriocidal effect on SA |
| | | | | | | | | | |
| 2 | Phenoxyethanol | none | Bacteriostatic | OK-28D | OK | OK | OK | OK | Bacteristatic effect on the five strains |
| | Phenoxyethanol | none | Bacteriocidal | KO | KO | OK | KO | KO | Bacteriocidal effect on SA |
| | | | | | | | | | |
| 3* | none | Capstone™ | Bacteriostatic | KO | OK | OK | OK | OK | Bacteriostaic effect on the five strains |
| | none | Capstone™ | Bacteriocidal | KO | KO | OK | OK | OK-28D | Bacteriocidal effect on SA and CA |
| | | | | | | | | | |
| 4 | Phenoxyethanol | Capstone™ | Bacteriostatic | OK | OK | OK | OK | OK | **Bacteriostatic effect on the five strains** |
| | Phenoxyethanol | Capstone™ | Bacteriocidal | OK | OK | OK | OK | OK | **Bacteriocidal effect on the five strains** |
| = no effect<br>: bacteristatic or bacteriocidal effect | | | | | | | | | |

EP 3 048 884 B1

**Claims**

1. An antimicrobial composition comprising:

    (a) at least a non ionic surfactant which is a fluoroalkyle polyethoxylated surfactant of formula $F(CF_2)_s\text{-}(CH_2\text{-}CH_2O)_r^H$ (VIII), wherein "s" is an integer and is equal or less than 10 and "r" is also an integer and is ranging from 1 to 16 , and
    (b) at least an alcohol compound of low molecular weight equal or less than 500 g/mol which is phenoxyethanol,

    whererin the weight ratio R of the non ionic surfactant/alcohol compound is such that $2.5 \leq R \leq 200$ and the content of the at least alcohol compound by weight, relative to the total weight of the antimicrobial composition is ranging from 0.01% to 5%.

2. The antimicrobial composition according to claim 1, wherein the weight ratio R of the non ionic surfactant/alcohol compound is such that $20 \leq R \leq 50$, preferably ratio R of the non ionic surfactant/alcohol compound is such that $20 \leq R \leq 40$.

3. The antimicrobial composition according to claim 1 or 2, wherein the content of the at least alcohol compound by weight, relative to the total weight of the antimicrobial composition is ranging from 0.25% to 2%, preferably between 0.5 to 1.5%.

4. The antimicrobial composition according to any one of the preceding claims, wherein 's" = 6 and "r" is ranging from 1 to 14 in the fluoroalkyle polyethoxylated surfactant of formula (VIII).

5. The antimicrobial composition according to any one of claims 1 to 4, wherein the content of the at least non ionic surfactant, by weight, relative to the total weight of the antimicrobial composition is ranging from 10 to 40 %

6. The antimicrobial composition according to claim 5, wherein the content of the at least non ionic surfactant, by weight, relative to the total weight of the antimicrobial composition is ranging from 20 to 30 %.

7. Aqueous solution comprising by weight relative to the total weight of the preparation at least 50% preferably 60%, of water and at least 10% of the antimicrobial composition according to any one of the preceding claims.

8. Dry microfiber tissue impregnated by an antimicrobial composition according to any one of claims 1 to 6, wherein said dry microfiber tissue comprises by weight, relative to the total weight of said dry microfiber tissue, less than 5 % of a solvent.

9. Dry microfiber tissue obtained by impregnation with an aqueous solution as defined in claim 7, of microfiber tissue comprising microfibers made of polymers comprising polyester units and polyamide units, followed by drying said microfiber tissue so as to remove solvents present in the aqueous solution, the dry microfiber tissue comprising, by weight, relative to the total weight of said dry microfiber tissue, less than 5 % of solvents.

10. Non woven wet tissue whose structure comprises a hydrophilic polymer, preferably a hydrophilic polymer comprising cellulosic units, and optionally a hydrophobic polymer, said tissue being impregnated by an aqueous solution as defined in claim 7.

11. Optical article comprising a substrate having at least one main surface coated with, a precursor coating of an antifog coating, **characterized in that** the coating precursor of the antifog coating

    - is obtained through the grafting of at least one compound having a polyoxyalkylene group,
    - and is further coated with a film obtained by applying onto said precursor coating temporary film comprising an antimicrobial composition as defined in any one of claims 1 to 6.

12. A method for imparting antimicrobial properties to an optical article having at least one main surface, comprising the application onto said main surface of a temporary antimicrobial film composition as defined in claim 11.

13. The method of claim 12, wherein the application onto said main surface comprises wiping said main surface with a tissue, whose structure comprises a hydrophilic polymer and a hydrophobic polymer, said tissue being impregnated with said temporary antimicrobial film composition.

**Patentansprüche**

1. Antimikrobielle Zusammensetzung, umfassend:

   (a) mindestens ein nichtionisches Tensid, bei dem es sich um ein polyethoxyliertes Fluoralkyltensid der Formel $F(CF_2)_s\text{-}(CH_2CH_2O)_rH$ (VIII) handelt, wobei "s" für eine ganze Zahl steht und gleich oder kleiner als 10 ist und "r" ebenfalls für eine ganze Zahl steht und im Bereich von 1 bis 16 liegt, und
   (b) mindestens eine Alkoholverbindung mit einem niedrigen Molekulargewicht gleich oder kleiner als 500 g/mol, bei der es sich um Phenoxyethanol handelt,

   wobei das Gewichtsverhältnis R nichtionisches Tensid/Alkoholverbindung $2{,}5{\leq}R{\leq}200$ beträgt und der Gehalt nach Gewicht an der mindestens einen Alkoholverbindung, bezogen auf das Gesamtgewicht der antimikrobiellen Zusammensetzung, im Bereich von 0,01% bis 5% liegt.

2. Antimikrobielle Zusammensetzung nach Anspruch 1, wobei das Gewichtsverhältnis R nichtionisches Tensid/Alkoholverbindung $20{\leq}R{\leq}50$ beträgt und das Verhältnis R nichtionisches Tensid/Alkoholverbindung vorzugsweise $20{\leq}R{\leq}40$ beträgt.

3. Antimikrobielle Zusammensetzung nach Anspruch 1 oder 2, wobei der Gehalt nach Gewicht an der mindestens einen Alkoholverbindung, bezogen auf das Gesamtgewicht der antimikrobiellen Zusammensetzung, im Bereich von 0,25% bis 2%, vorzugsweise zwischen 0,5 und 1,5%, liegt.

4. Antimikrobielle Zusammensetzung nach einem der vorhergehenden Ansprüche, wobei im polyethoxylierten Fluoralkyltensid der Formel (VIII) "s" = 6 und "r" im Bereich von 1 bis 14 liegt.

5. Antimikrobielle Zusammensetzung nach einem der Ansprüche 1 bis 4, wobei der Gehalt nach Gewicht an dem mindestens einen nichtionischen Tensid, bezogen auf das Gesamtgewicht der antimikrobiellen Zusammensetzung, im Bereich von 10 bis 40% liegt.

6. Antimikrobielle Zusammensetzung nach Anspruch 5, wobei der Gehalt nach Gewicht an dem mindestens einen nichtionischen Tensid, bezogen auf das Gesamtgewicht der antimikrobiellen Zusammensetzung, im Bereich von 20 bis 30% liegt.

7. Wässrige Lösung, umfassend, nach Gewicht, bezogen auf das Gesamtgewicht der Zubereitung, mindestens 50%, vorzugsweise 60%, Wasser und mindestens 10% an antimikrobieller Zusammensetzung nach einem der vorhergehenden Ansprüche.

8. Trockenes Mikrofasergewebe, imprägniert mit einer antimikrobiellen Zusammensetzung nach einem der Ansprüche 1 bis 6, wobei das trockene Mikrofasergewebe nach Gewicht, bezogen auf das Gesamtgewicht des trockenen Mikrofasergewebes, weniger als 5% an einem Lösungsmittel umfasst.

9. Trockenes Mikrofasergewebe, erhalten durch Imprägnieren mit einer wie in Anspruch 7 definierten wässrigen Lösung eines Mikrofasergewebes umfassend aus Polyestereinheiten und Polyamideinheiten umfassenden Polymeren hergestellte Mikrofasern, gefolgt vom Trocknen des Mikrofasergewebes zum Entfernen von in der wässrigen Lösung vorhandenen Lösungsmitteln, wobei das trockene Mikrofasergewebe nach Gewicht, bezogen auf das Gesamtgewicht des trockenen Mikrofasergewebes, weniger als 5% Lösungsmittel umfasst.

10. Vlies-Feuchtgewebe, dessen Struktur ein hydrophiles Polymer, vorzugsweise ein cellulosische Einheiten umfassendes hydrophiles Polymer, und gegebenenfalls ein hydrophobes Polymer umfasst, wobei das Gewebe mit einer wie in Anspruch 7 definierten wässrigen Lösung imprägniert ist.

11. Optisches Erzeugnis, umfassend ein Substrat mit mindestens einer Hauptoberfläche beschichtet mit einer Vorstufenbeschichtung einer Antibeschlagsbeschichtung, **dadurch gekennzeichnet, dass** die Beschichtungsvorstufe der Antibeschlagsbeschichtung

- durch Aufpfropfen mindestens einer Verbindung mit einer Polyoxyalkylengruppe erhalten wird
- und weiterhin mit einem Film beschichtet wird, der durch Aufbringen eines temporären Films auf die Vorstufenbeschichtung, umfassend eine wie in einem der Ansprüche 1 bis 6 definierte antimikrobielle Zusammensetzung, erhalten wird.

**12.** Verfahren zum Verleihen von antimikrobiellen Eigenschaften an ein optisches Erzeugnis mit mindestens einer Hauptoberfläche, umfassend das Aufbringen einer wie in Anspruch 11 definierten temporären antimikrobiellen Filmzusammensetzung auf die Hauptoberfläche.

**13.** Verfahren nach Anspruch 12, wobei das Aufbringen auf die Hauptoberfläche das Abwischen der Hauptoberfläche mit einem Gewebe, dessen Struktur ein hydrophiles Polymer und ein hydrophobes Polymer umfasst, wobei das Gewebe mit der temporären antimikrobiellen Filmzusammensetzung imprägniert ist, umfasst.

**Revendications**

**1.** Composition antimicrobienne comprenant :

(a) au moins un tensioactif non ionique qui est un tensioactif à base de fluoroalkyle polyéthoxylé de formule $F(CF_2)_s$-$(CH_2$-$CH_2O)H$ (VIII), dans laquelle "s" est un entier et est égal ou inférieur à 10 et "r" est également un entier et est dans la plage de 1 à 16, et
(b) au moins un composé alcoolique de poids moléculaire faible égal ou inférieur à 500 g/mol qui est le phénoxyéthanol,

dans laquelle le rapport en poids R du tensioactif non ionique/composé alcoolique est tel que $2,5 \leq R \leq 200$ et la teneur de l'au moins un composé alcoolique en poids, par rapport au poids total de la composition antimicrobienne, est dans la plage de 0,01 % à 5 %.

**2.** Composition antimicrobienne selon la revendication 1, dans laquelle le rapport en poids R du tensioactif non ionique/composé alcoolique est tel que $20 \leq R \leq 50$, de préférence le rapport R du tensioactif non ionique/composé alcoolique est tel que $20 \leq R \leq 40$.

**3.** Composition antimicrobienne selon la revendication 1 ou 2, dans laquelle la teneur de l'au moins un composé alcoolique en poids, par rapport au poids total de la composition antimicrobienne, est dans la plage de 0,25 % à 2 %, de préférence entre 0,5 et 1,5 %.

**4.** Composition antimicrobienne selon l'une quelconque des revendications précédentes, dans laquelle "s" = 6 et "r" est dans la plage de 1 à 14 dans le tensioactif à base de fluoroalkyle polyéthoxylé de formule (VIII).

**5.** Composition antimicrobienne selon l'une quelconque des revendications 1 à 4, dans laquelle la teneur de l'au moins un tensioactif non ionique, en poids, par rapport au poids total de la composition antimicrobienne est dans la plage de 10 à 40 %.

**6.** Composition antimicrobienne selon la revendication 5, dans laquelle la teneur de l'au moins un tensioactif non ionique, en poids, par rapport au poids total de la composition antimicrobienne est dans la plage de 20 à 30 %.

**7.** Solution aqueuse comprenant, en poids par rapport au poids total de la préparation, au moins 50 %, de préférence 60 %, d'eau et au moins 10 % de la composition antimicrobienne selon l'une quelconque des revendications précédentes.

**8.** Tissu de microfibres sec imprégné par une composition antimicrobienne selon l'une quelconque des revendications 1 à 6, ledit tissu de microfibres sec comprenant en poids, par rapport au poids total dudit tissu de microfibres sec, moins de 5 % d'un solvant.

**9.** Tissu de microfibres sec obtenu par imprégnation avec une solution aqueuse telle que définie dans la revendication 7, d'un tissu de microfibres comprenant des microfibres constituées de polymères comprenant des motifs de polyester et des motifs de polyamide, suivie du séchage dudit tissu de microfibres de façon à éliminer les solvants présents dans la solution aqueuse, le tissu de microfibres sec comprenant, en poids, par rapport au poids total dudit tissu de microfibres sec, moins de 5 % de solvants.

10. Tissu humide non tissé dont la structure comprend un polymère hydrophile, de préférence un polymère hydrophile comprenant des motifs cellulosiques, et facultativement un polymère hydrophobe, ledit tissu étant imprégné par une solution aqueuse telle que définie dans la revendication 7.

11. Article optique comprenant un substrat ayant au moins une surface principale revêtue avec un revêtement précurseur d'un revêtement antibuée, **caractérisé en ce que** le précurseur de revêtement du revêtement antibuée

    - est obtenu par greffage d'au moins un composé comportant un groupe polyoxyalkylène,
    - et est en outre revêtu avec un film obtenu par application sur ledit film temporaire de revêtement précurseur comprenant une composition antimicrobienne telle que définie dans l'une quelconque des revendications 1 à 6.

12. Procédé pour conférer des propriétés antimicrobiennes à un article optique comportant au moins une surface principale, comprenant l'application sur ladite surface principale d'une composition de film antimicrobien temporaire telle que définie dans la revendication 11.

13. Procédé selon la revendication 12, dans lequel l'application sur ladite surface principale comprend l'essuyage de ladite surface principale avec un tissu, dont la structure comprend un polymère hydrophile et un polymère hydrophobe, ledit tissu étant imprégné avec ladite composition de film antimicrobienne temporaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1324078 A
- EP 1275624 A
- WO 2011080472 A
- WO 980152 A
- WO 9414945 A
- WO 9617918 A
- US 5994286 A
- GB 1604859 A
- WO 2013013929 A
- US 2012019767 A
- US 3043697 A

### Non-patent literature cited in the description

- **W.C.GRIFFIN.** *J. Cosm Ploughshare. Chem.,* 1954, vol. 5, 249-56